# EUROPEAN PATENT SPECIFICATION

(11) **EP 3 049 140 B1**
(45) Date of publication and mention of the grant of the patent: **22.03.2017**
(21) Application number: 15771113.6
(22) Date of filing: 30.09.2015
(51) Int. Cl.: A61M 16/06

(54) **CUSHION ELEMENT FOR A PATIENT INTERFACE**
POLSTERELEMENT FÜR EINE PATIENTENSCHNITTSTELLE DER AKTUATOREN AUFWEIST
ÉLÉMENT D'AMORTISSEMENT DESTINÉ À UNE INTERFACE DE PATIENT QUI COMPREND DES ACTIONNEURS

(30) Priority: 02.10.2014 EP 14187465
(43) Date of publication of application: 03.08.2016
(73) Proprietor: Koninklijke Philips N.V., 5656 AE Eindhoven (NL)
(72) Inventor: VAN DE MOLENGRAAF, Roland Alexander, 5656 AE Eindhoven (NL); HENDRIKS, Cornelis Petrus, 5656 AE Eindhoven (NL)
(74) Representative: Schudelaro, Antonius Adrianus Petrus
(86) International application number: PCT/EP2015/072499
(87) International publication number: WO 2016/050814

(56) References cited:
- WO-A1-2010/016774
- WO-A1-2012/032437
- WO-A1-2013/183018
- WO-A1-2013/186650
- US-B1- 6 376 971

## Description

### FIELD OF THE INVENTION

The present invention relates to a cushion element for a patient interface for providing a flow of breathable gas to a patient. The present invention particularly relates to a cushion element that is improved with respect to a more efficient prevention of a formation of red marks within the face of the patient. Still further, the present invention relates to a patient interface and a pressure support system which make use of such a cushion element.

### BACKGROUND OF THE INVENTION

Patient interfaces, such as masks for covering the mouth and/or nose, are used for delivering gas to a patient. Such gases, like air, cleaned air, oxygen, or any modification of the latter, are submitted to the patient via the patient interface in a pressurized or unpressurized way.

For several chronic disorders and diseases, a long-term attachment of such a patient interface to a patient is necessary or at least advisable.

One non-limiting example for such a disease is obstructive sleep apnea or obstructive sleep apnea syndrome (OSA). OSA is usually caused by an obstruction of the upper airway. It is characterized by repetitive pauses in breathing during sleep and is usually associated with a reduction in blood oxygen saturation. These pauses in breathing, called apneas, typically last 20 to 40 seconds. The obstruction of the upper airway is usually caused by a reduced muscle tonus of the body that occurs during sleep. The human airway is composed of walls of soft tissue which can collapse and thereby obstruct breathing during sleep. Tongue tissue moves towards the back of the throat during sleep and thereby blocks the air passages. OSA is therefore commonly accompanied with snoring.

Different invasive and non-invasive treatments for OSA are known. One of the most powerful non-invasive treatments is the usage of Continuous Positive Airway Pressure (CPAP) or Bi-Positive Airway Pressure (BiPAP) in which a patient interface is connected to a pressure generator via a patient circuit including one or more tubes, wherein the pressure generator blows pressurized gas into the patient interface and into the patient's airway in order to keep it open. Positive air pressure is thus provided to a patient by means of the patient interface that is worn by the patient typically during sleep.

Examples for such patient interfaces are:
- nasal masks, which fit over the nose and deliver gas through the nasal passages,
- oral masks, which fit over the mouth and deliver gas through the mouth,
- full-face masks, which fit over both the nose and the mouth and deliver gas to both, and
- nasal pillows, which are regarded as patient interfaces as well within the scope of the present invention and which consist of small nasal inserts that deliver gas directly to the nasal passages.

The patient interface is usually positioned and donned to the patient's head using some kind of headgear. Further, the patient interface may comprise a forehead support. Such a forehead support is often designed as a pad that touches the forehead of a patient during use. It is often included in order to relief the pressure which the patient interface exerts onto the nose bridge.

Wearing a patient interface can be uncomfortable, since for providing an airtight seal between the patient interface and the patient's face, the patient interface has to be worn with a sufficient level of pressure on the face. It is thus evident that users of the patient interfaces experience a lot of disadvantages, wherein the most prominent disadvantage is the formation of facial red marks after a long-term usage of the patient interface. These red marks result from occlusions of blood vessels which arise from the pressure exerted by the patient interface.

A promising concept for preventing uncomfortable pressure points, red marks, indentations, and overall prolonged discomfort is the use of cushion elements that provide an alternating pressure onto the skin of the patient. This restores the skin blood flow in the depressurized part of the cushion element. One approach for providing cushion elements with an alternating pressure distribution is the use of electroactive polymers (EAPs) within the cushion elements. Dielectric elastomer actuators (DEAs) are smart material systems which produce large strains (up to 300%) and belong to the group of EAPs. Based on their simple working principle DEAs transform electric energy directly into mechanical work. DEAs are lightweight and free shapeable.

A DEA is a thin and flexible electroactive polymer sheet enclosed between two compliant electrodes. The thickness of the electroactive polymer sheet is controlled by the applied electrode voltage. Correspondingly, a thickness change results in elongation change. So both the thickness as well as the elongation of the sheet can be controlled. If applied to the skin, the alternating dimensions will impose an alternating pressure or stretch to the skin.

An example for a cushion element that includes EAP-actuators is known from WO 2013/183018 A1. The EAP-actuators used therein continuously alter the skin pressure distribution, provide a slow massaging motion to the skin, and relieve high local pressure peaks.

However, there is still room for improvement. One of the main technical challenges is the technical design and arrangement of such actuators within the cushion element. It is particularly challenging to design and arrange the actuators in such a way that static pressure points maybe effectively avoided.

A typical way to produce an EAP actuator is to spin coat or blade the polymer layer and to spray coat or paint the compliant electrode. There is a need to segment the electrodes so that alternating pressure (switching from segment A to segment B) can be realized. However, these electrode-segments are placed at a certain distance away from each other in order to prevent electric arcing from one electrode to the other. The gap between the electrode-segments is usually in the range of 1-2 mm and minimally in the range of 200-500µm. This gap can be responsible for static pressure points on the cushion element, which can lead to red marks.

### SUMMARY OF THE INVENTION

It is an object of the present invention to provide an improved cushion element for a patient interface, wherein the cushion element more effectively overcomes the problem of a red mark formation due to static pressure points on or within the cushion element. It is especially an object of the present invention to provide a cushion element including one or more actuators for providing an alternating pressure distribution within the cushion element, wherein the technical design and the arrangement of the actuators within the cushion element is improved.

According to an aspect of the present invention, a cushion element for a patient interface for providing a flow of breathable gas to a patient is presented, wherein the cushion element comprises:
- a face-contacting layer for contacting a face of the patient during use of the cushion element; and
- a responsive layer which is covered by the face-contacting layer;
wherein the responsive layer comprises a plurality of active and passive zones that are arranged alternately side by side to one another,
wherein each of the active zones comprises at least one actuator for moving the face-contacting layer, and wherein each of the passive zones comprises a cushion material that is arranged between the actuators,
wherein the face-contacting layer forms a corrugated or undulating surface covering both the active and the passive zones, wherein first parts of said surface covering the active zones project beyond or are recessed relative to second parts of said surface covering the passive zones.

According to a further aspect of the present invention, a patient interface comprising a cushion element of the above-mentioned type is presented.

According to still further aspect of the present invention, a pressure support system is presented which comprise a pressure generator for generating a flow of breathable gas, and a patient interface for providing the flow of breathable gas to a patient, wherein the patient interface comprises a cushion element of the above-mentioned type.

Preferred embodiments of the invention are defined in the dependent claims. It shall be understood that the claimed patient interface and the claimed pressure support system have similar and/or identical preferred embodiments as the claimed cushion element and as defined in the dependent claims.

The herein presented cushion element comprises a plurality of actuators which are configured to move the face-contacting layer of the cushion element in a predefined movement pattern in order to prevent a formation of red marks within the face of the patient. In contrast to prior art cushion elements of this type, the herein presented cushion element provides an improved arrangement of the actuators as well as an improved technical design of the cushion element itself.

The cushion element comprises a face-contacting layer which is configured to contact the face of the patient during use of the cushion element. This face-contacting layer builds the top surface of the cushion element. The face-contacting layer is preferably realized as a continuous layer that (fully) covers a responsive layer which is arranged below. The responsive layer is divided into so-called active and passive zones. However, it shall be noted that the terms "active" and "passive" shall not be considered to be limiting or to imply any special meaning other than the following. The active zones of the responsive layer are zones or areas in which at least one actuator is arranged. The passive zones are zones or areas in which a cushion material is arranged but no actuator. The active and passive zones could alternatively also be denoted as first and second zones. It shall be furthermore noted that the term "a plurality of" shall mean "at least two".

It is important that the active and passive zones are arranged alternately side by side to one another. This means that each active zone is sandwiched between two neighbouring passive zones, and each passive zone (except the passive zones arranged at the border of the cushion element) is sandwiched between two neighbouring active zones.

Since the actuators for moving the face-contacting layer are only arranged in the active zones, an actuation of the actuators will cause a direct movement of the active zones and the parts (herein denoted as "first parts") of the face-contacting layer covering and contacting the active zones. However, since the passive zones are sandwiched in between the active zones, an actuation of the actuators at least indirectly also causes a movement of the passive zones of the responsive layer and the parts (herein denoted as "second parts") of the face-contacting layer covering and contacting the passive zones. This indirect movement of the passive zones arises from the fact that cushion material, which may be present in the active zones between the electrodes of the actuators, is transferred to and from the passive zones due to material movement as soon as the actuators deform the active zones of the cushion element. It is therefore preferred that the cushion material comprises a pliable, resilient and/or elastic material that is deformable under pressure. Typical materials of this type are elastomers, like silicon, acrylics or polyurethane.

The one or more actuators that are arranged in each of the active zones of the cushion element are preferably configured to move the face-contacting layer in a periodic manner.

A further characterizing feature of the herein presented cushion element is the shape of the face-contacting layer. The face-contacting layer forms a corrugated or undulating surface which covers both the active and the passive zones. The first parts of said corrugated or undulating surface cover and contact the active zones, and the second parts of said corrugated and undulating surface cover and contact the passive zones of the responsive layer. The first and second parts of said surface are thus also arranged alternately side by side to one another (similar as the active and passive zones of the responsive layer). The first parts preferably also contact the neighbouring second parts, such that the corrugated or undulating surface is a continuously connected surface. The term "corrugated or undulating surface" shall mean that said surface is an uneven surface having elevations/bumps and recesses. The first parts of said surface, which cover the active zones, project beyond or are recessed relative to the second parts of said surface, which cover the passive zones. An activation of the actuators thus causes an undulating or wavelike movement of the face-contacting layer. This undulating or wavelike movement may be, but does not necessarily have to be a periodic movement. This movement prevents static pressure points and provides a smooth massaging effect in the face of the patient.

It shall be noted that the elevations and recesses of the face-contacting layer are not restricted to any special shape of their cross-sections. The elevations and recesses of the face-contacting layer may have any shape, like a cubical, rectangular, round, conical or elliptical shape.

According to an embodiment of the present invention, the actuators are configured to move the first parts of the corrugated or undulating surface relative to the second parts of said surface by expanding the active zones in a first direction towards the face of the patient and/or by contracting the active zones in a direction opposite the first direction. Thus, fairly simple and cost-saving actuators may be used which either contract or expand the cushion material within the active zones upon activation. The first direction is preferably meant to denote a direction transverse or perpendicular to the interface between the cushion element and the patient's face during use.

The active zones are preferably configured to communicate with the passive zones via the cushion material that is integrated in the responsive layer, such that an expansion of the active zones causes a contraction of the passive zones, and a contraction of the active zones causes an expansion of the passive zones. Depending on the movement of the active zones the cushion material is thus pushed from the active zones into the neighbouring passive zones or vice versa. If the actuators contract the active zones upon activation of the actuators, the cushion material present in the active zones in between the actuators will be pushed into the neighbouring passive zones. This will automatically cause the second parts of the face-contacting layer covering the passive zones to bulge outwardly towards the patient's face, whereas the first parts of the face-contacting layer covering the active zones will be pulled-in at the same time. A deactivation of the actuators would in this case cause the active zones to expand again, such that the cushion material will be pulled in the active zones from the neighbouring passive zones. This will cause the first parts of the face-contacting layer to bulge outwardly towards the patient's face, whereas the second parts of said layer will be pulled in. A periodic activation and deactivation of the actuators thus causes the corrugated or undulating surface of the face-contacting layer to move in an undulating or wave-like manner. The pressure points where the face-contacting layer touches the skin of the patient so to say switch places during this movement of the cushion element. The cushion element will alternately contact the patient's face with the first parts and the second parts of the face-contacting layer.

According to an embodiment, the active and passive zones are arranged in columns next to one another, wherein columns forming the active zones and columns forming the passive zones are arranged alternately side by side to one another. The columns forming the active zones and the columns forming the passive zones are preferably arranged parallel to one another and parallel to the first direction. It shall be noted again that this first direction denotes the direction of the movement of the active zones towards the patient's face, which movement is caused by the actuators. A parallel arrangement of the active and passive zones furthermore has the advantage that the switching pressure points are equally distributed over the cushion element. This results in a well-controllable and comfortable massaging effect of the patient's skin that prevents a formation of red marks. Leakages occurring at the interface between the patient's face and the cushion element are also prevented, since it is ensured that either the first parts or the second parts of the corrugated or undulating surface contact the skin and thereby provide a sufficient seal.

According to a further embodiment, the cushion element additionally comprises a support layer for providing mechanical stability to the responsive layer, wherein the support layer is arranged on a first side of the responsive layer, and wherein the face-contacting layer is arranged on a second side opposite the first side of the responsive layer. The responsive layer is in this embodiment thus sandwiched between the support layer and the face-contacting layer. It is especially preferred that each of the active zones and each of the passive zones contact the responsive layer with their first side and the face-contacting layer with their second side.

The additional support layer is preferably configured to be stiffer or less resilient than the face-contacting layer and/or the responsive layer of the cushion element. In this way it is ensured that the movement of the actuators is mainly translated into a movement of the face-contacting layer and not into a movement of the support layer.

According to a further embodiment, the at least one actuator of each active zone comprises an electroactive polymer and an electrode for activating the electroactive polymer. The electroactive polymer material may be the same as the before-mentioned cushion material. By the help of such electroactive polymer materials, electric energy may be directly transformed into mechanical work. Electroactive polymers provide the advantage that they are lightweight and free shapeable. By applying an alternating voltage to the electrodes the electroactive polymer material may be forced to expand and contract alternatingly. Examples of such electroactive polymer materials are: piezoelectric polymers, electromechanical polymers, relaxor ferroelectric polymers, electrostrictive polymers, dielectric elastomers, liquid crystal elastomers, conjugated polymers, Ionic Polymer Metal Composites, ionic gels, and polymer gels.

Even though the usage of such EAP actuators is preferred according to the present invention, the above-mentioned actuation and movement principle may also be accomplished with other types of actuators. Instead of EAPs, light-activated materials could e.g. be used as well. Other responsive materials, which could be used as actuators for the present invention, are: electroactive composites, electrostrictive ceramics or crystals, shape memory polymers, photomechanical materials, magnetostrictive materials, chemomechanical materials, and bimetal composites.

In order to amplify the movement of the face-contacting layer while still being able to use relatively low voltages (low energy), it is especially preferred that each of the active zones comprises a plurality of actuators. In case of the use of EAP actuators it is preferred that each of the plurality of actuators comprises an electroactive polymer and an electrode that is arranged transverse to the first direction. The electrodes are thus arranged above each other, wherein the gaps between the electrodes are filled up with the electroactive polymer material (the cushion material). Some electrodes may belong to two neighbouring actuators, whereas actuators arranged at the borders of the active zones belong to only one actuator. The plurality of actuators in each active zone may also be referred to as one common actuator arrangement.

According to a further embodiment, each of the active zones has a width (w) of 100µm ≤ w ≤ 100 mm, said width (w) being measured in a second direction transverse to the first direction. Said width dimensions of the active zones ensure a large enough but not too large movement of the first parts of the corrugated or undulating surface.

According to a further embodiment, the active zones are arranged in concentric closed loops. Such an arrangement is especially preferred if the cushion element is realized as a nose cushion or a mouth cushion. The passive zones will then similarly be arranged in concentric closed loops sandwiched between the active zones. This provides the advantage that even though the face-contacting layer alternatingly moves in the above-mentioned way during use, no pressure leakages occur, as the active and passive zones so to say fully surround the nose and/or the mouth of the patient. However, it shall be noted that such an arrangement is not necessarily needed if the cushion element is realized as a cushion or pad of a forehead support of the patient interface. Such forehead cushions usually do not have to form a seal.

### BRIEF DESCRIPTION OF THE DRAWINGS

These and other aspects of the invention will be apparent from and elucidated with reference to the embodiment described hereinafter. In the following drawings
Fig. 1 shows an exemplary embodiment of a patient interface in which a cushion element according to the present invention may be applied;
Fig. 2 shows a backside of the patient interface shown in Fig. 1; and
Fig. 3 shows a schematic cross section of an embodiment of the cushion element according to the present invention, wherein Fig. 3A shows the cushion element in a first operating state and Fig. 3B shows the cushion element in a second operating state.

### DETAILED DESCRIPTION OF THE INVENTION

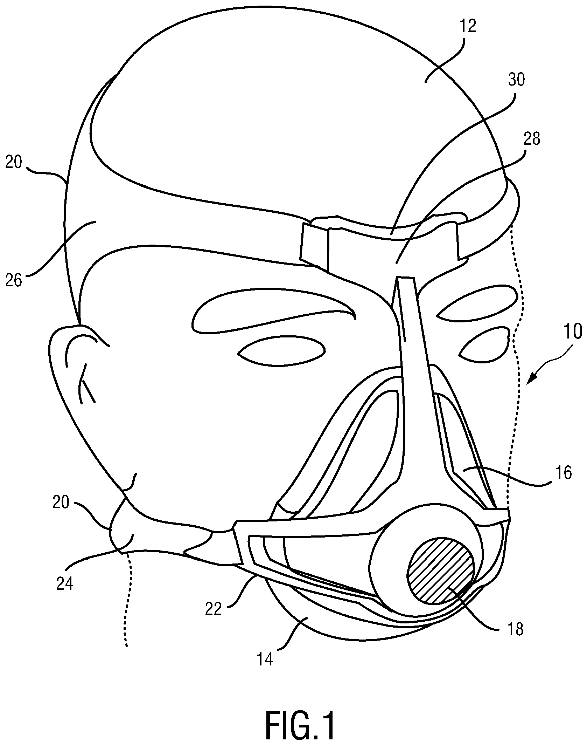

Fig. 1 shows an exemplary embodiment of a patient interface for delivering a flow of breathable gas to a patient. The patient interface is therein in its entirety denoted by reference numeral 10.

In this embodiment the patient interface 10 is designed as a full-face mask covering the mouth and the nose of a patient 12. It shall be noted that the patient interface 10 may alternatively be designed as a nose mask, a mouth mask or as a total face mask without leaving the scope of the present invention.

The patient interface 10 comprises a cushion element 14 and a mask shell 16. The cushion element 14 is designed to contact the face of the patient 12 and to provide an airtight seal at the interface between the patient's face and the patient interface 10. The cushion element 14 usually comprises a soft material, like silicon or any other rubber or suitable elastic material. The mask shell 16 provides a flexible, semi-rigid or rigid support structure for holding the cushion element 14. The mask shell 16 is usually connected to the backside of the cushion element 14, wherein the backside is meant to denote the side of the cushion element 14 opposite to the side of the cushion element 14 contacting the patient's face during use. The mask shell 16 may either be releasably or fixedly connected to the cushion element 14. The cushion element 14 and the mask shell 16 thus together form a cavity which is in this case designed to receive the mouth and the nose of the patient 12. It shall be noted that the cushion element 14 and the mask shell 16 may alternatively be formed as one integral piece.

On the opposite side directing away from the patient's face, the mask shell 16 preferably comprises a connector 18. Via this connector 18 the patient interface 10 may be connected to a hose (not shown) via which a pressurized flow of breathable gas can be submitted to the patient interface 10. The mask shell 16 is further connected to a headgear 20. This headgear 20 is used for attaching the patient interface 10 to the patient's head. According to the exemplary embodiment shown in Fig. 1, the headgear 20 comprises a rigid frame 22 and lower and upper headgear straps 24, 26. These lower and upper headgear straps 24, 26 may be connected to the frame 22 of the headgear 20 and used for donning the mask shell 16 and the cushion element 14 to the patient's face.

In the illustrated example the headgear 20 furthermore comprises a forehead support 28. This forehead support 28 allows stabilizing the patient interface 10 while being donned to the patient's face. The forehead support 28 reduces the pressure that is exerted onto the patient's nose during use. In order to make the forehead support 28 as comfortable as possible, the forehead support 28 furthermore comprises a forehead cushion 30 which is attached thereto. This forehead cushion 30 is according to the present invention also considered as a cushion element (similar as cushion element 14).

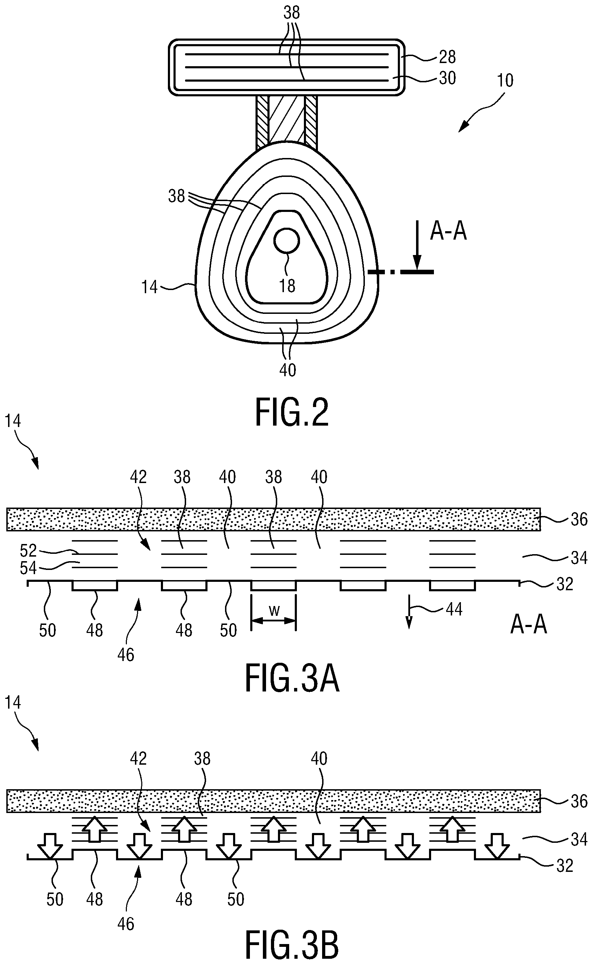

Fig. 2 shows a schematic view of the patient interface 10, the cushion element 14 and the forehead support 28 from the other side, i.e. from the side with which the cushion element 14 and the forehead cushion 30 contact the patient's face.

Figs. 3A and 3B show a schematic cross section of the cushion element 14 according to an embodiment of the present invention. The multi-layer structure of the cushion element 14 is therein illustrated in detail.

In the illustrated example the cushion element 14 basically comprises three layers: a face-contacting layer 32, a responsive layer 34 and a support layer 36. The face-contacting layer 32 forms the top surface of the cushion element 14 that contacts the patient's face during use. It may be made of a thin elastomeric film and has the function to provide a skin-friendly interface. The support layer 36 is arranged at the backside of the cushion element 14 that is usually connected to the mask shell 16. This support layer 36 is preferably made of a rubber material that is stiffer and less resilient than the materials from which the face-contacting layer 32 and the responsive layer 34 are made. The support layer 36 shall provide mechanical stability. However, it shall be noted that this support layer 36 is not necessarily needed if the function of providing mechanical stability is fulfilled by the mask shell 16 itself. The responsive layer 34 would in this case be sandwiched between the face-contacting layer 32 and the mask shell 16 instead of being sandwiched between the face-contacting layer 32 and the support layer 36 as shown in Fig. 3. Both alternatives are possible.

One of the central features of the presented cushion element 14 is the structure of the responsive layer 34. The responsive layer 34 is split up into active zones/areas 38 and passive zones/areas 40. Zones 38 are called active zones since these zones of the responsive layer 34 are configured to actively move the face-contacting layer 32. Zones 40 are denoted as passive zones since these zones are not actively moved (actuated), but move indirectly as soon as the active zones 38 are moved. This will become more apparent from the explanations given below.

As it can be seen in Figs. 3A and 3B, the active zones 38 and the passive zones 40 are arranged alternately side by side to one another. The active and passive zones 38, 40 are in the shown example arranged in parallel columns next to one another. Each active zone 38 comprises a plurality of actuators 42. These actuators 42 are configured to move the face-contacting layer 32 by expanding the active zones 38 in a first direction 44 towards the patient's face and/or by contracting the active zones 38 in a direction opposite the first direction 44. The actuators 42 are preferably configured to periodically expand and contract the active zones 38 of the responsive layer 34. In order to realize this movement the use of electrically actuated actuators 42 is preferred. Such actuators 42 may, for example, be controlled in such a way that the active zones 38 are contracted when the actuators 42 are activated, whereas the active zones 38 are expanded again when the actuators 42 are deactivated. However, it is also possible to control such actuators 42 the other way around, such that they expand the active zones 38 when being activated and contract the active zones 38 again when being deactivated. A preferred embodiment for such actuators 42 will be explained further below.

A further central feature of the cushion element 14 according to the present invention is the technical design and structure of the face-contacting layer 32. In contrast to most cushion elements 14 according to the prior art, this face-contacting layer 32 is not formed as an even, flat surface, but rather formed as an uneven, corrugated or undulating surface 46 (see Figs. 3A and 3B). This surface 46 comprises several interconnected segments which are herein denoted as first parts 48 and second parts 50 of the surface 46. The first parts 48 are the parts of the corrugated or undulating surface 46 that cover the active zones 38. The second parts 50 are the parts of the surface 46 that cover the passive zones 40. The first and second parts 48, 50 are therefore also arranged alternately side by side to one another. Depending on the activation state of the actuators 42, the first parts 48 of the surface 46 either project beyond or are recessed relative to the second parts 50 of the surface 46. In the exemplary embodiment shown in Fig. 3, Fig. 3A shows the deactivated state of the actuators 42 and Fig. 3B shows the activated state of the actuators 42. In this example, the first parts 48 thus project beyond the second parts 50 in the deactivated state (see Fig. 3A), whereas the first parts 48 are recessed relative to the second parts 50 in the activated state of the actuators 42 (see Fig. 3B). It is clear that the technical design may be chosen to be just the way around.

Due to the above-mentioned structure of the cushion element 14, the face-contacting layer 32 does not contact the patient's face along the whole surface 46 during use, but rather performs an undulating or wavelike movement. This prevents static pressure points which could otherwise lead to a formation of red marks within the patient's face.

In the preferred embodiment illustrated in Figs. 3A and 3B the actuators 42 are designed as electroactive polymer (EAP) actuators. In this case each EAP actuator 42 comprises two opposing electrodes 52 and an electroactive polymer material 54 that is arranged in between the electrodes 52. The electroactive polymer material 54 may be either configured to contract or to expand as soon as a voltage is applied to the electrodes 52. In the shown example, the electroactive polymer material 54 is configured to contract as soon as a voltage is applied thereto

It can be furthermore seen from Figs. 3A and 3B that each active zone 38 of the responsive layer 34 comprises a plurality of electrodes 52 and electroactive polymer layers 54 arranged in between them. The electrodes 52 are preferably arranged perpendicular to the first direction 44, i.e. parallel to the first parts 48 of the undulating surface 46.

Fig. 3B shows the activated state of the actuators 42, i.e. the state in which a voltage is applied to the electrodes 52. By comparing Fig. 3A with Fig. 3B it maybe seen that the first parts 48 of the undulating surface 46 so to say change places with the second parts 50 of the undulating surface 46 as soon as a voltage is applied to the electrodes 52 of the actuators 42. This results from the following fact: The active zones 38 communicate with the passive zones 40 via the cushion material that is integrated in the responsive layer 34. An activation of the actuators 42 causes the active zones 38 to contract (see Fig. 3B). This contraction shifts the material, which is present in between the electrodes 52, from the active zones 38 into the passive zones 40. The second parts 50 of the undulating surface 46 will then bulge outwardly so that they then project beyond the first parts 48 of the undulating 46. A wavelike movement of the face-contacting layer 32 may thus be achieved by alternately activating and deactivating the actuators 42 in the active zones 38 of the responsive layer 34. During this movement the first parts 48 and the second parts 50 of surface 46 will be alternately in contact with the patient's face.

The actuators 42 of the above-mentioned exemplary embodiment maybe easily driven by a voltage source (e.g. a battery) which is either integrated into the cushion element 14 itself or arranged at another position on the patient interface 10. This voltage source (not shown) is preferably connected to each of the actuators 42 via a voltage converter. A simple microprocessor maybe used to steer the actuators 42 in the above-mentioned way.

The width w of the active and passive zones 38, 40 maybe designed in a relatively free manner. Experiments of the applicant have shown that the best massaging effects may be achieved if the width w ranges between 100 µm and 100 mm.

It shall be further noted that the above-mentioned structure of the cushion element 14 may not only be used in the main cushion 14 forming the airtight seal of the mask 10, but could alternatively or additionally also be used in the forehead cushion 30. The only difference is that in the sealing cushion 14 one also needs to take account for the sealing behavior such a cushion element should fulfill. It is therefore preferred that the active zones 38 and passive zones 40 of the responsive layer 34 are arranged in concentric closed loops (see Fig. 2).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cushion element (14) for a patient interface (10) for providing a flow of breathable gas to a patient (12), wherein the cushion element (14) comprises:
- a face-contacting layer (32) for contacting a face of the patient (12) during use of the cushion element (14); and
- a responsive layer (34) which is covered by the face-contacting layer (32);
wherein the responsive layer (34) comprises a plurality of active and passive zones (3 8, 40) that are arranged alternately side by side to one another,
wherein each of the active zones (38) comprises at least one actuator (42) for moving the face-contacting layer (32), and wherein each of the passive zones (40) comprises a cushion material that is arranged between the actuators (42),
**characterized in that** the face-contacting layer (32) forms a corrugated or undulating surface (46) covering both the active and the passive zones (38,40), wherein first parts (48) of said surface (46) covering the active zones (38) project beyond or are recessed relative to second parts (50) of said surface (46) covering the passive zones (40).

2. The cushion element according to claim 1, wherein the actuators (42) are configured to move the first parts (48) of said surface (46) relative to the second parts (50) of said surface (46) by expanding the active zones (38) in a first direction (44) towards the face of the patient (12) and/or by contracting the active zones (38) in a direction opposite the first direction (44).

3. The cushion element according to claim 2, wherein the active zones (38) are configured to communicate with the passive zones (40) via the cushion material that is integrated in the responsive layer (34), such that an expansion of the active zones (38) causes a contraction of the passive zones (40), and a contraction of the active zones (38) causes an expansion of the passive zones (40).

4. The cushion element according to claim 1, wherein the active and passive zones (3 8, 40) are arranged in columns next to one another, wherein columns forming the active zones (38) and columns forming the passive zones (40) are arranged alternately side by side to one another.

5. The cushion element according to claims 2 and 4, wherein the columns forming the active zones (3 8) and the columns forming the passive zones (40) are arranged parallel to the first direction (44).

6. The cushion element according to claim 1, further comprising a support layer (36) for providing mechanical stability to the responsive layer (34), wherein the support layer (36) is arranged on a first side of the responsive layer (34), and wherein the face-contacting layer (32) is arranged on a second side opposite the first side of the responsive layer (34).

7. The cushion element according to claim 1, wherein the at least one actuator (42) of each active zone (38) comprises an electroactive polymer (54) and an electrode (52) for activating the electroactive polymer (54).

8. The cushion element according to claim 1, wherein each of the active zones (38) comprises a plurality of actuators (42).

9. The cushion element according to claims 2 and 8, wherein each of the plurality of actuators (42) comprises an electroactive polymer (54) and an electrode (52) that is arranged transverse to the first direction (44).

10. The cushion element according to claim 2, wherein each of the active zones (38) has a width (w) of 100µm ≤ w ≤ 100 mm, said width (w) being measured in a second direction transverse to the first direction (44).

11. The cushion element according to claim 1, wherein the plurality of active zones (38) are arranged in concentric closed loops.

12. The cushion element according to claim 1, wherein the cushion element (14) is one of a nose cushion, a mouth cushion and a forehead cushion (30).

13. A patient interface (10) for providing a flow of breathable gas to a patient (12), wherein the patient interface (10) comprises a cushion element (14) according to claim 1.

14. The patient interface according to claim 13, further comprising a voltage source which is connected to the at least one actuator (42) of each active zone.

15. A pressure support system, comprising:
- a pressure generator for generating a flow of breathable gas; and
- a patient interface (10) for providing the flow of breathable gas to a patient (12), wherein the patient interface (10) comprises a cushion element (14) according to claim 1.

## Patentansprüche

1. Polsterelement (14) für eine Patientenschnittstelle (10) zur Bereitstellung eines Stroms eines Atemgases für einen Patienten (12), wobei das Polsterelement (14) Folgendes umfasst:
- eine gesichtsberührende Schicht (32) zum Berühren des Gesichts eines Patienten (12) während der Verwendung des Polsterelements (14); und
- eine reaktive Schicht (34), die durch die gesichtsberührende Schicht (32) abgedeckt wird;
wobei die reaktive Schicht (34) eine Vielzahl von aktiven und passiven Zonen (38, 40) umfasst, die abwechselnd nebeneinander angeordnet ist,
wobei jede der aktiven Zonen (38) zumindest einen Aktuator (42) zum Bewegen der gesichtsberührenden Schicht (32) umfasst, und wobei jede der passiven Zonen (40) ein Polstermaterial umfasst, das zwischen den Aktuatoren (42) angeordnet ist,
**dadurch gekennzeichnet, dass** die gesichtsberührende Schicht (32) eine gerippte oder gewellte Fläche (46) bildet, die sowohl die aktive, als auch die passive Zone (38, 40) abdeckt, wobei erste Teile (48) der besagten Fläche (46), welche die aktiven Zonen (38) abdecken, über die zweiten Teile (50) der besagten Fläche (46), welche die passiven Zonen (40) abdecken, hinausragen oder im Verhältnis dazu vertieft sind.

2. Polsterelement nach Anspruch 1, wobei die Aktuatoren (42) konfiguriert sind, um die ersten Teile (48) der besagten Fläche (46) im Verhältnis zu den zweiten Teilen (50) der besagten Fläche (46) zu bewegen, indem sie die aktiven Zonen (38) in eine erste Richtung (44) zum Gesicht des Patienten (12) hin ausdehnen, und/ oder indem sie die aktiven Zonen (38) in eine entgegengesetzte Richtung zur ersten Richtung (44) einziehen.

3. Polsterelement nach Anspruch 2, wobei die aktiven Zonen (38) konfiguriert sind, um mit den passiven Zonen (40) über das Polstermaterial in Verbindung zu stehen, das in die reaktive Schicht (34) integriert ist, sodass eine Ausdehnung der aktiven Zonen (38) zum Einziehen der passiven Zonen (40) führt, und ein Einziehen der aktiven Zonen (38) zu einer Ausdehnung der passiven Zonen (40) führt.

4. Polsterelement nach Anspruch 1, wobei die aktive und die passive Zone (38, 40) in Spalten nebeneinander angeordnet sind, wobei die Spalten, welche die aktiven Zonen (38) bilden und die Spalten, welche die passiven Zonen (40) bilden, abwechselnd nebeneinander angeordnet sind.

5. Polsterelement nach den Ansprüchen 2 und 4, wobei die Spalten, welche die aktiven Zonen (38) bilden und die Spalten, welche die passiven Zonen (40) bilden, parallel zur ersten Richtung (44) angeordnet sind.

6. Polsterelement nach Anspruch 1, darüber hinaus eine Tragschicht (36) umfassend, die der reaktiven Schicht (34) mechanische Stabilität verleiht, wobei die Tragschicht (36) auf einer ersten Seite der reaktiven Schicht (34) angeordnet ist, und wobei die gesichtsberührende Schicht (32) auf einer zweiten Seite, gegenüber der ersten Seite der reaktiven Schicht (34) angeordnet ist.

7. Polsterelement nach Anspruch 1, wobei der zumindest eine Aktuator (42) einer jeden aktiven Zone (38) ein elektroaktives Polymer (54) und eine Elektrode (52) zum Aktivieren des elektroaktiven Polymers (54) umfasst.

8. Polsterelement nach Anspruch 1, wobei jede der aktiven Zonen (38) eine Vielzahl von Aktuatoren (42) umfasst.

9. Polsterelement nach den Ansprüchen 2 und 8, wobei jeder der Vielzahl von Aktuatoren (42) ein elektroaktives Polymer (54) und eine Elektrode (52) umfasst, die querlaufend zur ersten Richtung (44) angeordnet ist.

10. Polsterelement nach Anspruch 2, wobei jede der aktiven Zonen (38) eine Breite (w) von 100 µm ≤ w ≤ 100 mm aufweist, wobei die besagte Breite (w) in eine zweite Richtung gemessen wird, die querlaufend zur ersten Richtung (44) ist.

11. Polsterelement nach Anspruch 1, wobei die Vielzahl von aktiven Zonen (38) in konzentrischen geschlossenen Schleifen angeordnet sind.

12. Polsterelement nach Anspruch 1, wobei das Polsterelement (14) entweder ein Nasenpolster, ein Mundpolster oder ein Stirnpolster (30) ist.

13. Patientenschnittstelle (10) zur Bereitstellung eines Stroms eines Atemgases für einen Patienten (12), wobei die Patientenschnittstelle (10) ein Polsterelement (14) nach Anspruch 1 umfasst.

14. Patientenschnittstelle nach Anspruch 13, darüber hinaus eine Spannungsquelle umfassend, die mit dem zumindest einen Aktuator (42) einer jeden aktiven Zone verbunden ist.

15. Druckunterstützungssystem, Folgendes umfassend:
- einen Druckerzeuger zum Erzeugen eines Stroms von Atemgas; und
- eine Patientenschnittstelle (10) zur Bereitstellung eines Stroms eines Atemgases für einen Patienten (12), wobei die Patientenschnittstelle (10) ein Polsterelement (14) nach Anspruch 1 umfasst.

## Revendications

1. Elément coussinet (14) destiné à une interface de patient (10) pour fournir un flux de gaz respirable à un patient (12), dans lequel l'élément coussinet (14) comprend :
- une couche de contact avec le visage (32) pour entrer en contact avec le visage du patient (12) pendant l'utilisation de l'élément coussinet (14) ; et
- une couche répondante (34) qui est recouverte par la couche de contact avec le visage (32) ;
dans lequel la couche répondante (34) comprend une pluralité de zones actives et passives (38, 40) qui sont agencées en alternance les unes à côté des autres,
dans lequel chacune des zones actives (38) comprend au moins un actionneur (42) pour déplacer la couche de contact avec le visage (32), et dans lequel chacune des zones passives (40) comprend un matériau d'amortissement qui est agencé entre les actionneurs (42),
**caractérisé en ce que** la couche de contact avec le visage (32) forme une surface striée ou ondulée (46) recouvrant à la fois les zones actives et les zones passives (38, 40), dans lequel des premières parties (48) de ladite surface (46) recouvrant les zones actives (38) font saillie au-delà ou sont évidées par rapport à des secondes parties (50) de ladite surface (46) recouvrant les zones passives (40).

2. Elément coussinet selon la revendication 1, dans lequel les actionneurs (42) sont configurés pour déplacer les premières parties (48) de ladite surface (46) par rapport aux secondes parties (50) de ladite surface (46) en étendant les zones actives (38) dans une première direction (44) vers le visage du patient (12) et/ou en contractant les zones actives (38) dans une direction opposée à la première direction (44).

3. Elément coussinet selon la revendication 2, dans lequel les zones actives (38) sont configurées pour communiquer avec les zones passives (40) par le biais du matériau d'amortissement qui est intégré dans la couche répondante (34), de telle sorte qu'une expansion des zones actives (38) entraîne une contraction des zones passives (40), et qu'une contraction des zones actives (38) entraîne une expansion des zones passives (40).

4. Elément coussinet selon la revendication 1, dans lequel les zones actives et passives (38, 40) sont agencées en colonnes les unes à côté des autres, dans lequel des colonnes formant les zones actives (38) et des colonnes formant les zones passives (40) sont agencées en alternance les unes à côté des autres.

5. Elément coussinet selon les revendications 2 et 4, dans lequel les colonnes formant les zones actives (38) et les colonnes formant les zones passives (40) sont agencées parallèlement à la première direction (44).

6. Elément coussinet selon la revendication 1, comprenant en outre une couche de support (36) pour offrir une stabilité mécanique à la couche répondante (34), dans lequel la couche de support (36) est agencée sur un premier côté de la couche répondante (34), et dans lequel la couche de contact avec le visage (32) est agencée sur un second côté opposé au premier côté de la couche répondante (34).

7. Elément coussinet selon la revendication 1, dans lequel l'au moins un actionneur (42) de chaque zone active (38) comprend un polymère électroactif (54) et une électrode (52) pour activer le polymère électroactif (54).

8. Elément coussinet selon la revendication 1, dans lequel chacune des zones actives (38) comprend une pluralité d'actionneurs (42).

9. Elément coussinet selon les revendications 2 et 8, dans lequel chacun de la pluralité d'actionneurs (42) comprend un polymère électroactif (54) et une électrode (52) qui est agencée transversalement à la première direction (44).

10. Elément coussinet selon la revendication 2, dans lequel chacune des zones actives (38) a une largeur (w) de 100 µm ≤ w ≤ 100 mm, ladite largeur (w) étant mesurée dans une seconde direction transversale à la première direction (44).

11. Elément coussinet selon la revendication 1, dans lequel la pluralité de zones actives (38) sont agencées en boucles fermées concentriques.

12. Elément coussinet selon la revendication 1, dans lequel l'élément coussinet (14) est l'un parmi un coussinet nasal, un coussinet buccal et un coussinet frontal (30).

13. Interface de patient (10) pour fournir un flux d'air respirable à un patient (12), dans laquelle l'interface de patient (10) comprend un élément coussinet (14) selon la revendication 1.

14. Interface de patient selon la revendication 13, comprenant en outre une source de tension qui est connectée à l'au moins un actionneur (42) de chaque zone active.

15. Système de support de pression, comprenant :
- un générateur de pression pour générer un flux de gaz respirable ; et
- une interface de patient (10) pour fournir le flux de gaz respirable à un patient (12), dans lequel l'interface de patient (10) comprend un élément coussinet (14) selon la revendication 1
